# EUROPEAN PATENT SPECIFICATION

(11) **EP 2 371 268 B1**
(45) Date of publication and mention of the grant of the patent: **04.05.2016**
(21) Application number: 08878888.0
(22) Date of filing: 15.12.2008
(51) Int. Cl.: A61B 1/06, G02B 23/26

(54) **ILLUMINATION DEVICE AND ENDOSCOPIC APPARATUS**
BELEUCHTUNGSVORRICHTUNG UND ENDOSKOPISCHES GERÄT
DISPOSITIF D'ÉCLAIRAGE ET APPAREIL ENDOSCOPIQUE

(43) Date of publication of application: 05.10.2011
(73) Proprietor: Olympus Corporation, Shibuya-ku Tokyo 151-0072 (JP)
(72) Inventor: KOBAYASHI, Eiichi, Tokyo 151-0072 (JP)
(74) Representative: Schicker, Silvia
(86) International application number: PCT/JP2008/072781
(87) International publication number: WO 2010/070720

(56) References cited:
- EP-A1- 2 359 739
- WO-A1-2006/038502
- WO-A1-2010/064322
- JP-A- 2008 023 262
- JP-A- 2008 284 030
- JP-A- 2008 289 712
- JP-A- 2008 301 873
- JP-A- 2008 307 171
- US-A1- 2008 283 770

## Description

### Technical Field

The present invention relates to an endoscope apparatus comprising an illumination device.

### Background Art

Heretofore, in order to observe the inside of a subject, such as the inside of a mechanical structure in an industrial field or the inside of the body of a patient in a medical field, an endoscope apparatus is widely used. The endoscope apparatus has an insertion portion which is inserted into the subject, and is provided with an observation portion at the front end of the insertion portion. Accordingly, the endoscope apparatus can observe the inside of the subject. Meanwhile, there are many cases where the inside of the subject which is observed by the endoscope apparatus has insufficient brightness at the time of observation by the observation portion. For this reason, an illumination device which illuminates the inside of the subject is embedded in the endoscope apparatus.

As the illumination device, an illumination device is suggested which includes the following three constituent elements (for example, see Patent Documents 1 and 2): a light source unit which is provided on the base end side of the insertion portion to emit laser light as excitation light; a light guide which is provided from the base end to the front end in the insertion portion to guide laser light emitted from the light source; and a fluorescent member which is provided at the front end of the insertion portion to emit illumination light with laser light guided by the light guide as excitation light.
[Patent Document 1] Japanese Patent Application, First Publication No. 2006-26135
[Patent Document 2] Japanese Patent Application, First Publication No. 2006-288535

US 2008/283770 A1 discloses an illumination light detecting optical system to be used for an optical apparatus which comprises a light emitting element which emits excitation light, an insertion part of a long-narrow-shape, a fluorescence luminescence component arranged near a top of the insertion part, an excitation light guiding means which leads the excitation light emitted from the light emitting element to the fluorescence luminescence component, and a light detecting means which detects return light which is a part of the illumination light.

JP 2008-284030 discloses an illumination light detecting optical system including a light emitting element for emitting excitation light, an insertion part, a fluorescent member being disposed near a distal end of the insertion part, an excitation light guiding means for guiding the excitation light from the light emitting element to the fluorescent member, and a light detection means for detecting return light of part of the illumination light.

JP 2008-289712 discloses an illuminating apparatus comprising a light source for emitting excitation light, a light source driving section for supplying power to the light source, the fluorescent member for emitting illuminating light in response to the excitation light, a light transmitting member for introducing the excitation light to the fluorescent member, a light detecting means for detecting a light volume of the excitation light or the illuminating light, a control section for sensing whether the light volume is abnormal or not on the basis of the detection result of the light detecting means, and a regulating means for keeping the light source in such a condition that emission of excitation light is stopped when a light volume abnormality signal is output once from the control section.

EP 2359739 A1 (published after the filing date of the present document) discloses an illumination device including a light source section outputting excitation light, a fluorescent member excited by the excitation light to emit illumination light, a first light transmitting section disposed between the light source section and the fluorescent member to guide the excitation light output from the light source section to the fluorescent member, and a second light transmitting section, which is arranged closer to the distal side than the fluorescent member to guide the illumination light emitted from the fluorescent member.

### SUMMARY OF THE INVENTION

### Disclosure of Invention

### Technical Problem

However, in the endoscope apparatus of Patent Documents 1 and 2, when any one of the light source unit, the light guide, and the fluorescent member of the embedded illumination device is deteriorated or damaged, the subject may not be observed accurately.

Accordingly, there is demand for an illumination device and an endoscope apparatus capable of quantitatively evaluating the amount of illumination light accurately.

### Technical Solution

The present invention is defined in claim 1.

An illumination device according to an aspect of the invention includes a light source unit which emits excitation light, a first optical transmission unit which guides excitation light from the light source unit, a fluorescent member which is excited by excitation light to emit illumination light, and a light determination unit which determines the amount of illumination light emitted from the fluorescent member to output a determination signal.

According to the illumination device of the aspect of the invention, excitation light is emitted from the light source unit and guided by the first optical transmission unit to excite the fluorescent member, such that illumination light can be emitted from the fluorescent member and can illuminate the subject. At this time, illumination light is determined by the light determination unit, thereby quantitatively evaluating the amount of illumination light. The light determination unit is provided near the fluorescent member, such that illumination light can be determined in a state of being not attenuated immediately after being emitted from the fluorescent member, thereby evaluating the amount of illumination light accurately.

The illumination device of the aspect further includes a second optical transmission unit which guides illumination light from the fluorescent member. The light determination unit may determine the amount of illumination light guided by the second optical transmission unit.

The illumination device of the aspect further includes an excitation light determination unit which determines the amount of excitation light emitted from the light source unit or the amount of excitation light guided by the first optical transmission unit.

The illumination device of the aspect may further include a case which substantially has a tubular shape to accommodate the fluorescent member inside and is configured such that excitation light is input from a base end portion and illumination light is output from a front end portion. For this reason, the light determination unit may determine a part of illumination light which leaks from determination ports formed in the case to the outside.

According to the illumination device of the aspect, the fluorescent member is excited inside the case, such that illumination light to be emitted can be output from the front end portion without being diffused. The determination ports are formed in the case, such that the light determination unit determines a part of illumination light leaking from the determination ports, thereby efficiently determining the amount of illumination light.

In the illumination device of the aspect, the determination ports may be formed in the lateral surface of the case in the emission direction of illumination light.

According to the illumination device of the aspect, the determination ports are formed in the lateral surface of the case in the emission direction of illumination light, such that the amount of illumination light can be determined by the light determination unit without limiting the output range of illumination light in the front end portion.

In the illumination device of the aspect, the fluorescent member may be formed by mixing a fluorescent material which produces fluorescence by excitation light and a light scattering material which scatters excitation light. For this reason, the determination ports of the case may be formed in the lateral surface which is in contact with the fluorescent member.

According to the illumination device of the aspect, the fluorescent member is formed by mixing the fluorescent material and the light scattering material, such that illumination light which is generated when the fluorescent material is excited can be scattered by the light scattering material. For this reason, light can be input to the determination ports formed at positions of the lateral surface in contact with the fluorescent member.

The illumination device of the aspect may further include an elongated insertion portion which is inserted into a subject, a reflection portion for reflecting illumination light leaking from the determination ports to the outside in an axial direction of the insertion portion, and a guide rod which guides illumination light reflected by the reflection portion in the axial direction. For this reason, the light determination unit may be at a position different from the fluorescent member in the axial direction to determine illumination light guided by the guide rod.

According to the illumination device of the aspect, illumination light which leaks from the determination ports to the outside is reflected by the reflection portion, is guided by the guide rod in the axial direction of the insertion portion, and is determined by the light determination unit. The light determination unit is at a position different from the fluorescent member in the axial direction of the insertion portion, making it possible to reduce the diameter of the insertion portion.

The illumination device of the aspect may further include an amplifier which is provided near the fluorescent member to amplify and transmit the determination signal output from the light determination unit.

According to the illumination device of the aspect, the determination signal from the light determination unit is amplified by the amplifier inside the insertion portion, making it possible to suppress degradation in output and an increase in noise in the elongated insertion portion and to allow the transmission of the determination signal to the base end side.

In the illumination device of the aspect, the light determination unit may have a plurality of light sensors which separately determine the amount of illumination light in different wavelength regions.

According to the illumination device of the aspect, the amount of illumination light is separately determined in different wavelength regions by a plurality of light sensors of the light determination unit, thereby evaluating the state of illumination light in more detail. Simultaneously, when an abnormality is confirmed, it is possible to specify the reason for the abnormality in more detail.

In the illumination device of the aspect, the light determination unit has a first light sensor which determines the amount of light in the illumination light substantially having the same wavelength as the wavelength of excitation light emitted from the light source unit.

According to the illumination device of the aspect, the amount of light in the illumination light substantially having the same wavelength component as excitation light is determined by the first light sensor of the light determination unit, thereby evaluating the following two points in more detail: whether or not excitation light is irradiated from the light source unit onto the fluorescent member in a desired amount; and whether or not excitation light passes through the fluorescent member and is irradiated outside.

In the illumination device of the aspect, the light determination unit has a second light sensor which determines the amount of light in the illumination light having a wavelength other than the wavelength of excitation light emitted from the light source unit.

According to the illumination device of the aspect, the amount of light in the illumination light having a component other than the wavelength of excitation light is determined by the second light sensor of the light determination unit. Thus, it is possible to evaluate in more detail whether or not illumination light is appropriately excited by excitation light and output in a desired amount.

In the illumination device of the aspect, the second optical transmission unit is provided on more front end side than the fluorescent member.

According to the illumination device of the aspect, illumination light emitted from the fluorescent member is guided toward the front end side by the second optical transmission unit and is irradiated to the outside. The position where the fluorescent member is provided can be freely set in accordance with the length of the second optical transmission unit. That is, the position where the fluorescent member is provided can be set such that the heat release condition is satisfactory and the fluorescent member is not easily damaged due to external force.

In the illumination device of the aspect, the first optical transmission unit may be a light guide.

According to the illumination device of the aspect, excitation light emitted from the light source unit is appropriately guided by the light guide serving as the first optical transmission unit and irradiated onto the fluorescent member. Thus, the fluorescent member is excited such that illumination light can be emitted and irradiated to the outside.

An endoscope apparatus of the aspect of the invention may include the above-described illumination device which further includes an elongated insertion portion, which is inserted into a subject, and observation portion provided at the front end of the insertion portion to observe the inside of the subject.

In the illumination device of the aspect, the light determination unit and the excitation light determination unit are provided. The excitation light determination unit is provided on at least the base end side of the light source unit near the light source unit, or the front end side of the light source unit near the light source unit.

According to the illumination device of the aspect, the determination results of the amount of light obtained from the light determination unit and the excitation light determination unit provided at multiple places are compared with each other. Thus, it is possible to diagnose the deterioration state of the illumination device or the constituent members of the illumination device.

For example, the excitation light determination unit is provided near the light source unit and on the front end side of the light source unit, such that it is possible to determine excitation light in a state of being not attenuated immediately after being emitted from the light source unit, making it possible to evaluate the amount of excitation light accurately. In this case, the excitation light determination unit may be provided near the light source unit and on the base end side of the light source unit.

When the excitation light determination unit is further provided near the fluorescent member and on the base end side of the fluorescent member, the determination results of the amount of light by the excitation light determination units provided at two places are compared with each other. Thus, it is possible to diagnose the deterioration state of the first optical transmission unit which is interposed between the excitation light determination units.

When the light determination unit is further provided on the base end side of the second optical transmission unit near the fluorescent member and on the front end side of the second optical transmission unit, the following effect is obtained. That is, the determination results of the amount of light by the light determination unit provided on the base and side and the front end side of the second optical transmission unit are compared with each other, such that it is possible to diagnose the deterioration state of the second optical transmission unit which is interposed between the light determination units.

When the excitation light determination unit and the light determination unit are respectively provided on the base end side and the front end side near the fluorescent member, the following effect is obtained. That is, the determination results of the amount of light by the excitation light determination unit and the light determination unit provided at two places are compared with each other, such that it is possible to diagnose the deterioration state of the fluorescent member which is interposed between the excitation light determination unit and the light determination unit.

When the excitation light determination unit is provided on the front end side near the light source unit, and the light determination unit is provided on the front end side near the fluorescent member, the following effect is obtained. That is, the determination results of the amount of light by the excitation light determination unit and the light determination unit provided at two places are compared with each other, such that it is possible to compare the amount of excitation light emitted from the light source with the amount of illumination light emitted from the fluorescent member.

The excitation light determination unit provided near the light source unit and a video output to the monitor are compared with each other, such that it is possible to diagnose the deterioration state of the entire illumination device.

In the illumination device, when the fluorescent material in the fluorescent member is distributed so as to be segregated on the front end side, in the fluorescent member, a base end-side portion having a relatively low content ratio of the fluorescent material may be used as the first optical transmission unit. That is, in case where the fluorescent member has a fluorescent material so as to segregate in resin, in the resin, a portion having a low content ratio of the fluorescent material may be regarded as the first optical transmission unit.

In this case, the first optical transmission unit is inexpensive compared to a fiber made of quartz or the like. Even when the first optical transmission unit is damaged by external stress, resin having a Young's modulus lower than quartz is used as the first optical transmission unit, such that the resin is deformed, thereby suppressing damage to the first optical transmission unit.

In the illumination device of the aspect, the light source unit may be an LED.

In this case, in particular, it is possible to detect degradation in translucency due to deterioration of sealing resin constituting an LED, and in comparing between an LED and a halogen lamp, an LED is inexpensive and has a long lifetime. With the use of an LED, it is possible to reduce the complexity of temperature management compared to the related art.

According to the endoscope apparatus of the axspect of the invention, it is possible to quantitatively evaluate the amount of illumination light by the illumination device accurately and to accurately observe the subject by the observation portion on the basis of stable illumination.

### Advantageous Effects

According to the aspect of the illumination device and the endoscope apparatus of the invention, the light determination unit and the excitation light determination unit are provided. For this reason, when excitation light is irradiated from the light source unit onto the fluorescent member such that illumination light is emitted from the fluorescent member and is irradiated to the outside, it is possible to quantitatively evaluate the amount of illumination light accurately.

### Brief Description of Drawings

FIG. 1 is an overall conceptual diagram showing the external configuration of an endoscope apparatus according to a first example useful to understand the invention.
FIG. 2 is an overall conceptual diagram showing the internal configuration of the endoscope apparatus according to the first example.
FIG. 3 is a sectional view showing the details of a front end portion and a bending portion in an insertion portion of the endoscope apparatus according to the first example.
FIG. 4 is a front view of a front end portion in the insertion portion of the endoscope apparatus according to the first example.
FIG. 5 is a sectional view showing the details of an illumination light generation unit in the endoscope apparatus according to the first example.
FIG. 6 is a sectional view taken along the line A-A of FIG. 5.
FIG. 7 is a sectional view showing the details of an illumination light generation unit in an endoscope apparatus according to a first modification of the first example.
FIG. 8 is a sectional view showing the details of an illumination light generation unit in an endoscope apparatus according to a second modification of the first example.
FIG. 9 is a sectional view taken along the line B-B of FIG. 8.
FIG. 10 is an overall conceptual diagram showing the internal configuration of an endoscope apparatus according to a second example, which is an embodiment of the invention.
FIG. 11 is a sectional view showing the details of a front end portion and a bending portion in an insertion portion of the endoscope apparatus according to the second example.
FIG. 12 is a sectional view showing the details of a bending portion and a flexible tube portion in an insertion portion of the endoscope apparatus according to the second example.
FIG. 13 is a sectional view showing the details of an illumination light generation unit in an endoscope apparatus according to the second example.
FIG. 14 is a sectional view showing the details of an illumination light generation unit in an endoscope apparatus according to a first modification of the second example.
FIG. 15 is a sectional view taken along the line C-C of FIG. 14.
FIG. 16 is a sectional view showing the details of an illumination light generation unit in an endoscope apparatus according to a second modification of the second example.
FIG. 17 is a sectional view taken along the line D-D of FIG. 16.
FIG. 18 is a schematic view illustrating a modification of a fluorescent member and a first optical transmission unit.

### Explanation of References

1, 50: endoscope apparatus
1a, 50a: illumination device
2: insertion portion
5: observation portion
20: illumination portion
21: laser diode (light source unit)
22, 41: fluorescent member
41a, 80: fluorescent material
41b: light scattering material
24: light guide (first light guide)
26, 55: case
26d, 55d: lateral surface
26e, 55e: determination port
30: light determination unit
30a: first light sensor (light sensor)
30b: second light sensor (light sensor)
31a, 31b: amplifier
46: prism (reflection portion)
47: guide rod
53: first light guide (first optical transmission unit)
54: second light guide (second optical transmission unit)
60: excitation light determination unit
60a: first light sensor (light sensor)
60b: second light sensor (light sensor)
70: resin

### Best Mode for Carrying out the Invention

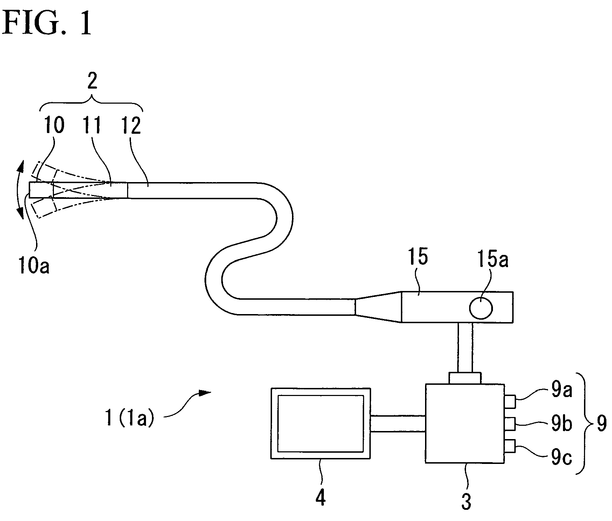

An example useful to understand the invention will be described with reference to FIGS. 1 to 6.

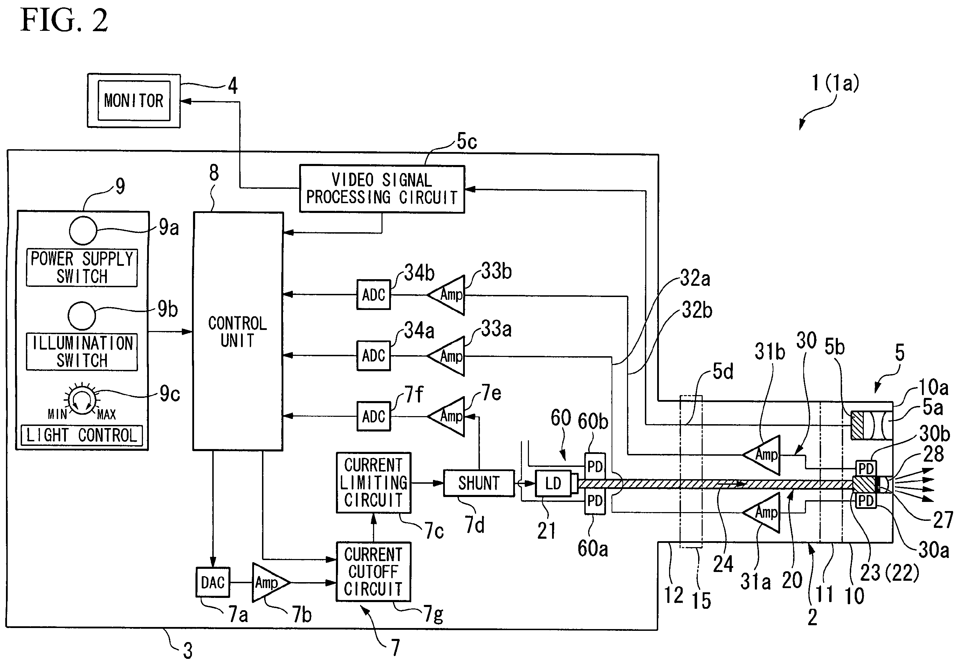

As shown in FIGS. 1 and 2, an endoscope apparatus 1 includes an elongated insertion portion 2 which is inserted into a subject, an apparatus main body 3 which is provided on the base end side of the insertion portion 2, and a monitor 4 which is connected to the apparatus main body 3. The insertion portion 2 and the apparatus main body 3 are provided with an observation portion 5 that observes the subject on the front end side of the insertion portion 2, and an illumination portion 20 that illuminates the subject to be observed by the observation portion 5. Thus, the endoscope apparatus 1 includes an illumination device 1a which has the insertion portion 2, the illumination portion 20, and a control unit 8 described below. Hereinafter, the details of the configuration will be described.

### (First Example)

A first example useful to understand the invention will be described with reference to FIGS. 1 to 6.

As shown in FIGS. 1 and 2, an endoscope apparatus 1 of this embodiment includes an elongated insertion portion 2 which is inserted into a subject, an apparatus main body 3 which is provided on the base end side of the insertion portion 2, and a monitor 4 which is connected to the apparatus main body 3. The insertion portion 2 and the apparatus main body 3 are provided with an observation portion 5 that observes the subject on the front end side of the insertion portion 2, and an illumination portion 20 that illuminates the subject to be observed by the observation portion 5. Thus, the endoscope apparatus 1 includes an illumination device 1a which has the insertion portion 2, the illumination portion 20, and a control unit 8 described below. Hereinafter, the details of the configuration will be described.

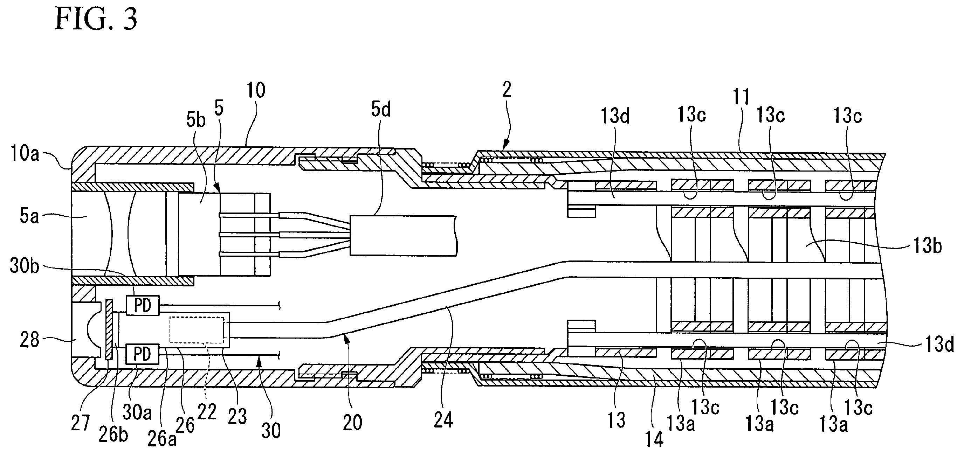

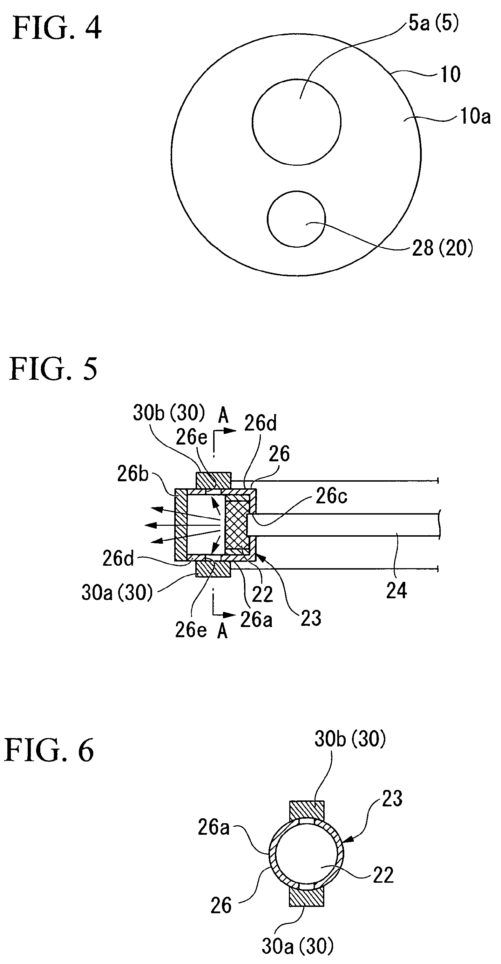

As shown in FIG. 1, the insertion portion 2 is a soft type which has, in order from the front end, a hard front end portion 10, a bending portion 11 configured to be freely bent by a bending operating portion 15 described below, and a flexible tube portion 12 configured to be bent in accordance with the shape of the subject. As shown in FIG. 3, the front end portion 10 is substantially formed in a tubular shape having a front end surface 10a, and an objective optical system 5a of the observation portion 5 and an illumination optical system 28 of the illumination portion 20 described below are provided so as to be exposed from the front end surface 10a. As shown in FIG. 4, the flexible tube portion 12 is a flexible member which substantially has a long tubular shape.

As shown in FIG. 3, the bending portion 11 has a bending tube 13 which is constituted by connecting a plurality of bending pieces 13a, and an elastically deformable elastic tubular member 14 which substantially has a tubular shape and is provided so as to cover the outer circumference of the bending tube 13. The bending tube 13 of the bending portion 11 is fixed to the front end portion 10 on the front end side and, though not shown, is fixed to the flexible tube portion 12 on the base end side. In each bending piece 13a constituting the bending tube 13, a pair of convex portions 13b (in FIG. 3, only one convex portion is shown) are formed to protrude in an arc shape toward the base end side at two opposing places in the radial direction, and are in contact with the front ends of adjacent bending pieces 13a. The positions of the convex portions 13b of each bending piece 13a are set to be substantially the same in the circumferential direction. For this reason, each bending piece 13a substantially rotates in the same direction around a pair of convex portions 13b in a state of being provided inside the elastic tubular member 14, such that the bending tube 13 can be bent in the corresponding direction as a whole. In each bending piece 13a, a pair of through holes 13c are formed at a position corresponding to the direction being bent as the bending tube 13, that is, at an intermediate position of a pair of convex portions 13b, and a pair of operating wires 13d are respectively inserted thereinto. In a pair of operating wires 13d, the front end side is fixed to the front end of the bending tube 13, and the base end side is inserted into the flexible tube portion 12, and as shown in FIG. 1, is connected to the bending operating portion 15 provided at the base end of the flexible tube portion 12. A joystick 15a is provided in the bending operating portion 15, and one of a pair of operating wires 13d can be dragged by the operation of the joystick 15a. Thus, the bending portion 11 can be bent toward the dragged operating wire 13d as a whole.

As shown in FIGS. 2 and 3, the observation portion 5 has an objective optical system 5a, a CCD (Charge Coupled Device) 5b, a video signal processing circuit 5c, and a signal cable 5d. The objective optical system 5a is provided to be exposed in the front end portion 10 of the insertion portion 2. The CCD 5b is provided at the imaging position of the objective optical system 5a inside the front end portion 10. The video signal processing circuit 5c is embedded in the apparatus main body 3. The signal cable 5d is provided in the insertion portion 2 to connect the CCD 5b and the video signal processing circuit 5c. An observation image of the subject imaged by the objective optical system 5a is converted to an electrical signal by the CCD 5b and transmitted by the signal cable 5d as an image signal. The video signal processing circuit 5c generates a video signal on the basis of the transmitted image signal and outputs the video signal to the monitor 4 connected to the apparatus main body 3, such that the video signal can be viewed as video.

The illumination portion 20 has a laser diode 21, an illumination light generation unit 23, and a light guide (first optical transmission unit) 24. The laser diode 21 is a light source unit which is embedded in the apparatus main body 3 to emit laser light as excitation light. The illumination light generation unit 23 is provided inside the front end portion 10 of the insertion portion 2, and has a fluorescent member 22. The light guide 24 is provided between the laser diode 21 and the illumination light generation unit 23 inside the insertion portion 2. The laser diode 21 can emit single-color laser light in a light amount according to the magnitude of a current to be supplied, and in this embodiment, can emit blue laser light.

As shown in FIGS. 5 and 6, the illumination light generation unit 23 has the fluorescent member 22 and a case 26 which substantially has a tubular shape to accommodate the fluorescent member 22 inside. The fluorescent member 22 is formed of a fluorescent material 80 (not shown) which is excited by laser light to emit white light as illumination light. The case 26 has a case main body 26a which substantially has a tubular shape with the front end side opened and in which the fluorescent member 22 is accommodated, and a cover glass 26b which closes the opening on the front end side of the case main body 26a. A connection port 26c to which the front end of the light guide 24 is connected is provided on the base end side of the case main body 26a, such that laser light from the laser diode 21 guided by the light guide 24 can be irradiated onto the internal fluorescent member 22. Thus, illumination light which is excited by laser light and emitted from the fluorescent member 22 is irradiated from the inside of the case main body 26a to the outside through the cover glass 26b.

As shown in FIG. 3, the illumination portion 20 has a diffuser plate 27 which is formed at the front end of the illumination light generation unit 23 inside the front end portion 10 of the insertion portion 2, and an illumination optical system 28 which is provided on the front end side of the diffuser plate 27 and exposed from a front end surface 10a of the front end portion 10. The diffuser plate 27 is, for example, a glass plate which is subjected to surface roughening or includes a granular reflector, and is configured to diffuse and transmit illumination light emitted from the illumination light generation unit 23. The illumination optical system 28 focuses and shapes illumination light having passed through the diffuser plate 27, and irradiates illumination light to the outside.

As shown in FIG. 2, the apparatus main body 3 is embedded with a light source driving unit 7 which supplies a current to the laser diode 21, and a control unit 8 which controls the amount of a current supplied from the light source driving unit 7. A control panel 9 is connected to the control unit 8. The control panel 9 is provided with a power supply switch 9a, an illumination switch 9b, and an illumination knob 9c. The power supply switch 9a turns on or off the power supply of the entire apparatus. The illumination switch 9b turns on or off illumination light by the illumination portion 20. The illumination knob 9c adjusts the amount of illumination light in a state where the illumination switch 9b is turned on. When the power supply switch 9a is turned on, the turning on or off of illumination by the illumination portion 20 and the adjustment of the light amount can be manually carried out through the control unit 8 by an operation of the control panel 9.

The light source driving unit 7 has a DA converter 7a, an amplifier 7b, and a current limiting circuit 7c. The DA converter 7a DA-converts a current command value output from the control unit 8. The amplifier 7b amplifies the current command value DA-converted by the DA converter 7a. The current limiting circuit 7c supplies a current to the laser diode 21 in a corresponding amount of current on the basis of the amplified current command value. The laser diode 21 emits laser light in a light amount according to the current command value (the amount of current). A shunt 7d serving as current determination portion is interposed between the current limiting circuit 7c and the laser diode 21. The shunt 7d determines the amount of current supplied from the current limiting circuit 7c to the laser diode 21 and outputs the determined amount of current as a determination signal. The output determination signal is input to the control unit 8 through the amplifier 7e and the AD converter 7f, and the control unit 8 performs feedback control on the basis of the determined amount of current. A current cutoff circuit 7g is interposed between the current limiting circuit 7c and the amplifier 7b, and the control unit 8 can output a cutoff signal to the current cutoff circuit 7g. For this reason, the current cutoff circuit 7g can cut off the input of the current command value to the current limiting circuit 7c on the basis of the cutoff signal to stop the supply of a current to the laser diode 21.

As shown in FIG. 2, the insertion portion 2 and the apparatus main body 3 are provided with a light determination unit 30 which determines the amount of illumination light emitted from the fluorescent member 22 in the illumination portion 20 and outputs a determination signal. Specifically, the light determination unit 30 is provided near the fluorescent member 22 inside the insertion portion 2 and has a first light sensor 30a and a second light sensor 30b. The first light sensor 30a is a photodiode which determines the amount of light in the illumination light substantially having the same wavelength as laser light. The second light sensor 30b is a photodiode which determines the amount of light in the illumination light having a wavelength other than the wavelength of laser light. As shown in FIGS. 5 and 6, the first light sensor 30a and the second light sensor 30b of the light determination unit 30 are arranged as follows. That is, the arrangement is made to determine a part of illumination light which leaks from determination ports 26e, which are formed to communicate from the outside to the inside of the case 26 in a lateral surface 26d of the case 26 of the illumination light generation unit 23 in the emission direction of illumination light, to the outside. Determination signals output from the first light sensor 30a and the second light sensor 30b are respectively amplified by amplifiers 31a and 31b which are provided to be close to each other on the base end side of the bending portion 11 inside the insertion portion 2. Thereafter, the determination signals are respectively transmitted through signal lines 32a and 32b provided in the insertion portion 2, and are further amplified by amplifiers 33a and 33b inside the apparatus main body 3. Simultaneously, the determination signals are respectively AD-converted by AD converters 34a and 34b and input to the control unit 8.

Next, the action of the endoscope apparatus 1 will be described. As shown in FIGS. 1 and 2, if the power supply switch 9a and the illumination switch 9b of the control panel 9 are turned on, the control unit 8 outputs a current command value corresponding to the illumination knob 9c to the light source driving unit 7. Thereafter, the light source driving unit 7 supplies a current having a magnitude corresponding to the input current command value to the laser diode 21. For this reason, the laser diode 21 emits laser light in a light amount according to the amount of current to be supplied, and laser light is guided toward the front end side by the light guide 24 and irradiated onto the fluorescent member 22 to excite the fluorescent member 22. Thus, the fluorescent member 22 emits illumination light in a light amount according to the amount of laser light. As shown in FIGS. 5 and 6, most illumination light emitted from the fluorescent member 22 is input directly to the cover glass 26b located on the front end side or is reflected by the case 26 and input to the cover glass 26b. For this reason, illumination light is emitted toward the front end side without being diffused. The emitted illumination light is shaped by the illumination optical system 28 and is irradiated to the outside. Thus, an image of the inside of the subject can be appropriately received by the observation portion 5 using reflected light of illumination light, and the insertion portion 2 is inserted into the subject while confirming an observation image displayed on the monitor 4, such that detailed observation can be carried out. At the time of observation, the joystick 15a of the bending operating portion 15 is operated to bend the bending portion 11 of the insertion portion 2 in a predetermined direction. Therefore, it is possible to adjust the direction of the objective optical system 5a of the observation portion 5 to observe a wider area of the inside of the subject. At this time, the diffuser plate 27 is provided at the front end of the light guide 24, such that illumination light is further diffused and is irradiated to the outside. Accordingly, a wider area of the inside of the object is effectively irradiated and a wider area of the inside of the object can be observed.

As shown in FIGS. 5 and 6, the determination ports 26e are formed in a part of the case 26, and a part of illumination light emitted from the fluorescent member 22, excluding illumination light which is input to the cover glass 26b and illuminates the outside, is input to the determination ports 26e. For this reason, the amount of illumination light can be efficiently determined by the first light sensor 30a and the second light sensor 30b of the light determination unit 30 provided in the determination ports 26e. In particular, since the determination ports 26e are formed in the lateral surface 26d of the case 26, there is no case where the range in which light is input to the light guide 25 is limited.

Meanwhile, the determination results of the light determination unit 30 are input to the control unit 8. For this reason, the control unit 8 can quantitatively evaluate the amount of illumination light on the basis of the determination results and can detect the presence or absence of deterioration in or damage to the fluorescent member 22. The control unit 8 can detect the presence or absence of deterioration in or damage to the laser diode 21 or the first light guide 24, and can constantly observe the subject by the observation portion 5 accurately on the basis of stable illumination.

In particular, the light determination unit 30 includes the first light sensor 30a and the second light sensor 30b. The amount of light in the illumination light substantially having the same wavelength as laser light is determined by the first light sensor 30a, such that the following two points can be evaluated in more detail: whether or not laser light is irradiated from the laser diode 21 onto the fluorescent member 22 in a desired light amount; and whether or not laser light passes through the fluorescent member 22 and is irradiated outside. The amount of light having a component other than the wavelength of laser light is determined by the second light sensor 30b, such that it is possible to evaluate in more detail whether or not illumination light is appropriately excited by laser light and output in a desired light amount. The light determination unit 30 is provided near the fluorescent member 22 to determine illumination light, thereby determining illumination light in a state of being not attenuated immediately after being emitted from the fluorescent member 22 and accurately evaluating the amount of illumination light. The determination results of the light determination unit 30 are respectively amplified by the amplifiers 31a and 31b inside the insertion portion 2, transmitted to the apparatus main body 3 through the signal lines 32a and 32b, and input to the control unit 8. Therefore, even in the elongated insertion portion 2, it is possible to suppress degradation in output and an increase in noise and to transmit the determination signals of the light determination unit 30 to the base end side. It is also possible to more accurately detect the occurrence of abnormality and to specify the reason for the abnormality by the control unit 8.

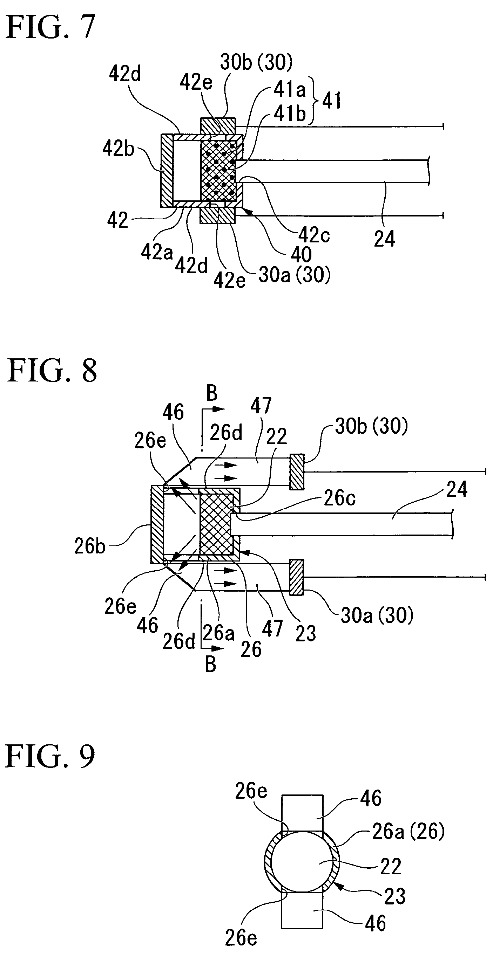

FIG. 7 shows a first modification of this embodiment. As shown in FIG 7, in an endoscope apparatus of this modification, an illumination light generation unit 40 has a fluorescent member 41 and a case 42 which substantially has a tubular shape and accommodates the fluorescent member 41. The fluorescent member 41 is formed by mixing a fluorescent material 41a which is excited by laser light to emit illumination light and a light scattering material 41b which scatters laser light. The case 42 has a case main body 42a which substantially has a tubular shape with the front end side opened, and a cover glass 42b which closes the opening on the front end side of the case main body 42a. In the case main body 42a, a connection port 42c is formed such that the front end of the light guide 24 is connected to the base end side thereof, and determination ports 42e are formed in a lateral surface 42d to communicate between the inside and the outside of the case main body 42a. Specifically, the determination ports 42e are formed at a position of the lateral surface 42d in contact with the fluorescent member 41.

In this modification, if laser light guided by the light guide 24 is irradiated onto the fluorescent member 41, the fluorescent material 41a forming the fluorescent member 41 is excited to emit illumination light. The light scattering material 41b is mixed in the fluorescent member 41, such that the generated illumination light is scattered by the light scattering material 41b. For this reason, it is possible to efficiently input illumination light to the determination ports 42e located laterally to the fluorescent member 41 and to effectively determine the amount of illumination light by the first light sensor 30a and the second light sensor 30b of the light determination unit 30.

FIGS. 8 and 9 show a second modification of this embodiment. As shown in FIGS. 8 and 9, an endoscope apparatus of this modification includes prisms 46 and guide rods 47. The prisms 46 are reflection portion respectively provided in the determination ports 26e of the case 26. The guide rods 47 are respectively optically connected to the prisms 46, and the first light sensor 30a and the second light sensor 30b of the light determination unit 30 are respectively connected to the base ends of the guide rods 47. The prisms 46 are provided to reflect illumination light, which is input from the inside of the case main body 26a to the determination ports 26e, toward the base end side of the insertion portion 2 in the axial direction. The guide rods 47 are provided to extend from the prisms 46 toward the base end side in the axial direction of the insertion portion 2 to guide illumination light reflected by the prisms 46 toward the base end side. For this reason, the guide rods 47 can input illumination light to the first light sensor 30a and the second light sensor 30b of the light determination unit 30.

In this modification, illumination light which is reflected by the prisms 46 and guided by the guide rods 47 can be determined by the light determination unit 30. For this reason, the first light sensor 30a and the second light sensor 30b of the light determination unit 30 are at positions different from the illumination light generation unit 23 in the axial direction of the insertion portion 2. Thus, in the insertion portion 2, it is possible to achieve the reduction in the diameter of the front end portion 10 in which the illumination light generation unit 23 and the light determination unit 30 are provided.

### (Second Example, Embodiment of the invention)

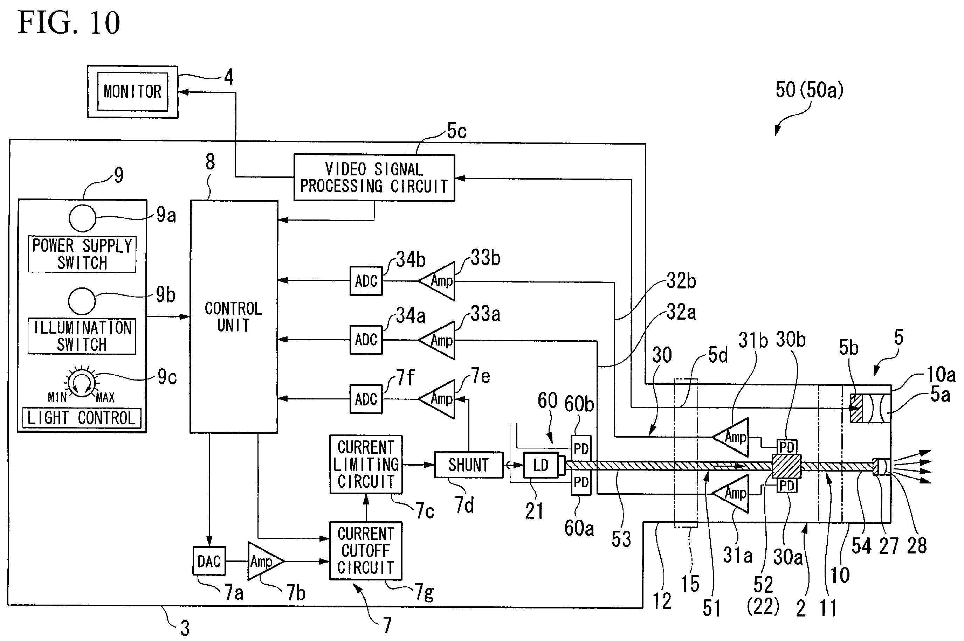

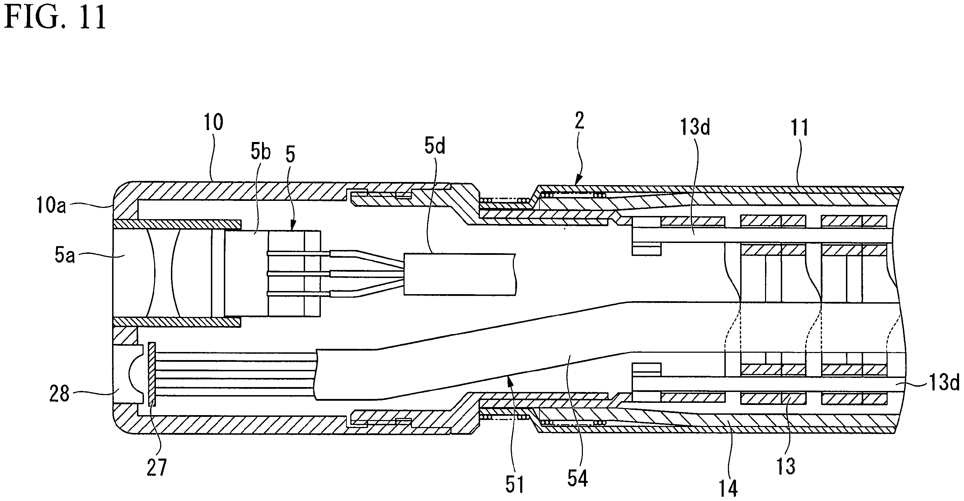

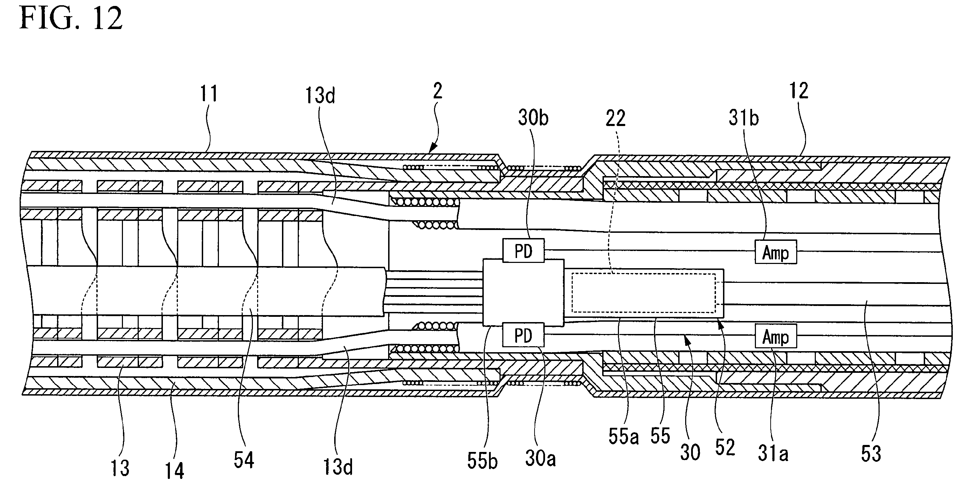

Next, an embodiment of the invention will be described. FIGS. 10 to 13 show the second example, which is an embodiment of the invention. In this embodiment, the same members as the members used in the above-described first example are represented by the same reference numerals, and a description thereof will be omitted.

As shown in FIGS. 10 to 12, in an endoscope apparatus 50 of this embodiment, illumination portion 51 of an illumination device 50a has a laser diode 21, an illumination light generation unit 52 having a fluorescent member 22, a first light guide 53, and a second light guide 54. The first light guide 53 is a first optical transmission unit which is provided between the laser diode 21 and the illumination light generation unit 52. The second light guide 54 is a first optical transmission unit which is provided between the laser diode 21 and the illumination light generation unit 52. The diffuser plate 27 and the illumination optical system 28 are provided on the front end side of the second light guide 54.

In this embodiment, the first light guide 53 is a single-core fiber, and the second light guide 54 is a multicore fiber. The illumination light generation unit 52 is arranged at a position near the bending portion 11 on more base end side than the bending portion 11, that is, at the front end inside the flexible tube portion 12, and has the fluorescent member 22 and a case 55 which accommodates the fluorescent member 22 inside. The case 55 has a case main body 55a in which the fluorescent member 22 is accommodated, and a cap 55b which is externally fitted into the front end side of the case main body 55a. A connection port 55c to which the front end of the first light guide 53 is connected is provided on the base end side of the case main body 55a. Thus, laser light from the laser diode 21 guided by the first light guide 53 can be irradiated onto the internal fluorescent member 22. The front end side of the case main body 55a is opened and communicates with the cap 55b. The front end side of the cap 55b is externally fitted into the base end of the second light guide 54. For this reason, illumination light which is excited by laser light and emitted from the fluorescent member 22 is input to the base end of the second light guide 54 through the inside of the cap 55b and guided toward the front end side. Illumination light passes through the diffuser plate 27 and the illumination optical system 28, and is irradiated to the outside.

According to the endoscope apparatus of this embodiment, the second light guide 54 is provided, such that the position where the fluorescent member 22 is provided can be freely set in accordance with the length of the second light guide 54. For this reason, the fluorescent member 22 is not provided in the front end portion 10 which is substantially at the same position as the CCD 5b in the axial direction of the insertion portion 2. Thus, since there is no case where the CCD 5b is influenced by heat from the fluorescent member 22, it is possible to reduce the occurrence of noise from the CCD 5b. The fluorescent member 22 is provided in the flexible tube portion 12 having a small number of embedded components, such that the heat release condition of the fluorescent member 22 can be appropriately set. For this reason, it is possible to suppress deterioration of the fluorescent member 22 and degradation in conversion efficiency of illumination light from laser light. The first light sensor 30a and the second light sensor 30b of the light determination unit 30 can be more easily arranged near the fluorescent member 22.

In this embodiment, the fluorescent member 22 is located in the illumination portion 51, and the fluorescent member 22 and the first light guide 53 are located on more base end side than the bending portion 11. For this reason, when the insertion portion 2 is inserted into the subjected, even if the front end portion 10 or the bending portion 11 located on the front end side in the insertion portion 2 is damaged, there is no case where the first light guide 53 or the fluorescent member 22 is damaged due to damage to the front end portion 10 or the bending portion 11. Thus, there is no case where the first light guide 53 or the fluorescent member 22 is damaged, and laser light emitted from the laser diode 21 leaks from the damaged portion to the outside until laser light is irradiated onto the fluorescent member 22 and affects the subject.

Meanwhile, when the bending portion 11 or the front end portion 10 is damaged, the second light guide 54, the diffuser plate 27, or the illumination optical system 28 may be damaged. However, since the second light guide 54, the diffuser plate 27, and the illumination optical system 28 are provided on more front end side than the fluorescent member 22, illumination light merely leaks to the outside, and there is no case where leakage light affects the subject. The fluorescent member 22 is provided near the bending portion 11, such that the fluorescent member 22 is arranged as close to the front end side as possible in the range on more base end side than the bending portion 11. For this reason, it is possible to minimize the length of the second light guide 54 which guides illumination light, thus minimizing the attenuation of illumination light guided by the second light guide 54.

In this embodiment, the first optical transmission unit and the second optical transmission unit are constituted by two different light guides of the first light guide 53 and the second light guide 54.

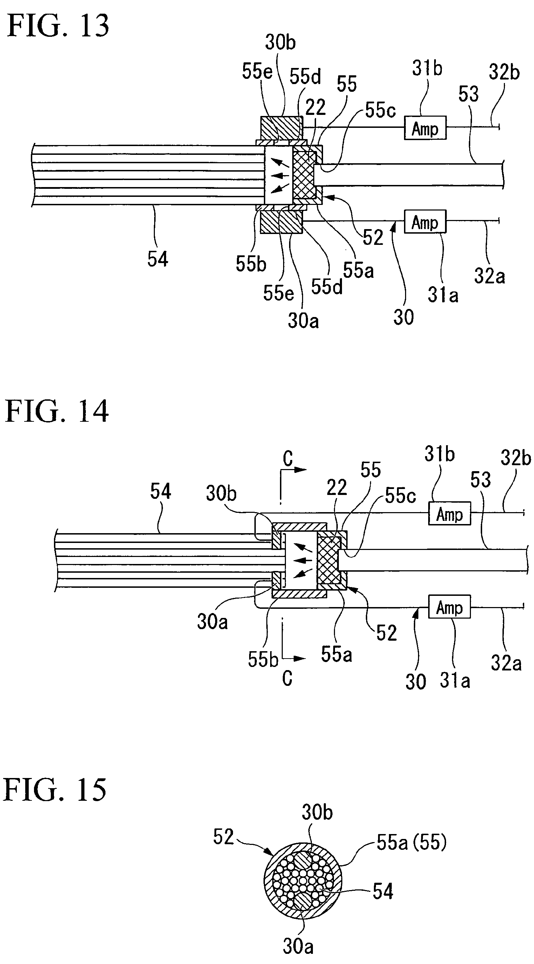

FIGS. 14 and 15 show a first modification of this embodiment. As shown in FIGS. 14 and 15, in this modification, in the cap 55b of the case 55, part of the opening on the front end side into which the base end of the second light guide 54 is fitted is provided as a determination port. The first light sensor 30a and the second light sensor 30b of the light determination unit 30 are fitted into the determination port. For this reason, illumination light emitted from the fluorescent member 22 is input to the second light guide 54 directly or after being reflected by the case 55, and is determined by the light determination unit 30.

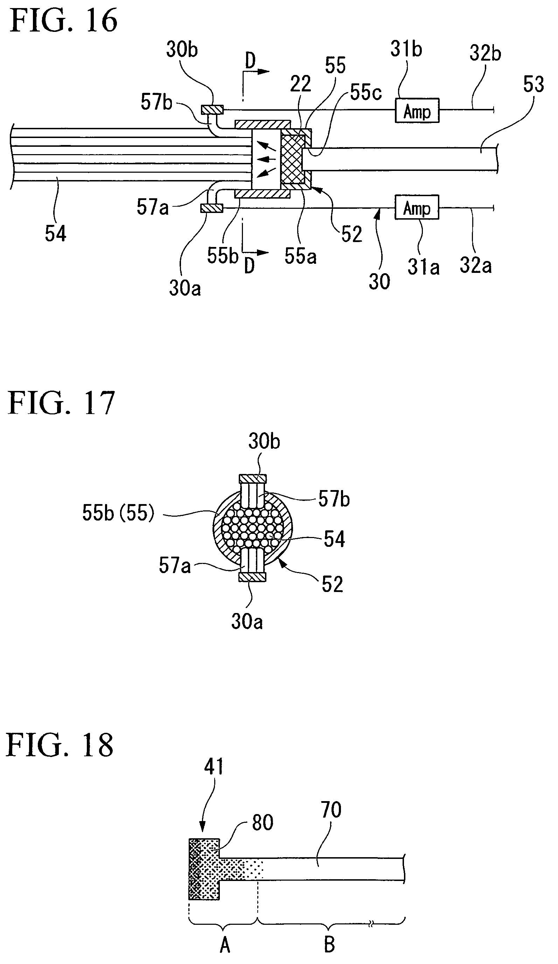

As shown in FIGS. 16 and 17, in this modification, in the cap 55b of the case 55, part of the opening on the front end side into which the base end of the second light guide 54 is fitted is provided as a determination port, and the base ends of fiber bundles 57a and 57b are fitted into the determination port. Simultaneously, the first light sensor 30a and the second light sensor 30b of the light determination unit 30 are respectively optically connected to the front ends of the fiber bundles 57a and 57b. For this reason, illumination light emitted from the fluorescent member 22 is input to the second light guide 54 directly or after being reflected by the case 55. Simultaneously, illumination light is input to the fiber bundles 57a and 57b, and determined by the first light sensor 30a and the second light sensor 30b of the light determination unit 30.

Although the embodiments of the invention have been described in detail with reference to the drawings, a specific configuration is not limited to the embodiments, and design changes may be made without departing from the scope of the invention.

Although in the foregoing embodiments, a case has been described where the insertion portion 2 is a soft type having the flexible tube portion 12, the invention is not limited thereto, a hard type having a hard tube, instead of the flexible tube portion 12, may be used. Although a case has been described where the laser diode of the illumination portion is embedded in the apparatus main body 3, the invention is not limited thereto. For example, the laser diode may be embedded in the bending operating portion 15 on the base end side of the insertion portion 2. The bending operating portion 15 may not be provided at the base end of the insertion portion 2, and may be connected to the apparatus main body 3 separately from the insertion portion 2.

In this case, the laser diode may be provided inside the apparatus main body 3 or inside the insertion portion 2 on the base end side.

Although a case has been described where the light determination unit includes the first light sensor and the second light sensor, the invention is not limited thereto. For example, either the first light sensor or the second light sensor may be provided, or the amount of illumination light may be determined separately in three or more wavelength regions by three of more light sensors. The amount of illumination light over the entire wavelength region may be determined by a single light sensor. The state of illumination light can be evaluated by at least one light sensor, and if a plurality of light sensors are provided, the state of illumination light can be evaluated in more detail. Simultaneously, when abnormality is confirmed, it becomes possible to specify the reason for abnormality in more detail. Although in the foregoing embodiments, a case has been described where the light determination unit determines the amount of illumination light, the light determination unit may determine the amount of laser light emitted from the laser diode.

In the illumination device 1a or 50a, the light determination unit 30 may be used to determine the amount of illumination light guided by the second optical transmission unit 54, instead of the amount of illumination light emitted from the fluorescent member 22 or 41.

Thus, the position where the light determination unit 30 is provided is not limited to near the fluorescent member 22 or 41, and may be provided on the front end side of the fluorescent member 22 or 41 near the fluorescent member 22 or 41, or the front end side of the second optical transmission unit 54.

The illumination device 1a or 50a further includes an excitation light determination unit 60 which determines the amount of excitation light emitted from the light source unit 21.

The excitation light determination unit 60 is provided near the laser diode 21, and has a first light sensor 60a and a second light sensor 60b. The first light sensor 60a is a photodiode which determines the amount of light substantially having the same wavelength as laser light in excitation light. The second light sensor 60b is a photodiode which determines the amount of light having a wavelength other than the wavelength of laser light in excitation light. Other detailed configurations of the excitation light determination unit 60 are based on the detailed configurations of the light determination unit 30, thus a detailed description thereof will be omitted. That is, the determination signals output from the first light sensor 60a and the second light sensor 60b are amplified by the amplifiers (not shown). Thereafter, the determination signals are transmitted through the signal lines (not shown). The determination signals are amplified by the amplifiers (not show), AD-converted by the AD converters (not shown), and input to the control unit 8.

The excitation light determination unit 60 is provided on the base end side of the light source unit 21 near the light source unit 21 or on the front end side of the light source unit 21 near the light source unit 21.

In the illumination device 1a or 50a according to the embodiment of the invention, a light determination unit 30 and excitation light determination unit 60 are provided. It is possible to diagnose the deterioration state of the illumination device 1a, 50a, or the constituent members of the illumination device 1a or 50a on the basis of the obtained determination results of the light amount.

For example, the excitation light determination unit 60 is provided on the front end side of the light source unit 21 near the light source unit 21, such that it is possible to determine excitation light in a state of being not attenuated immediately after being emitted from the light source unit 21. For this reason, it is possible to accurately evaluate the amount of excitation light. In this case, the excitation light determination unit 60 may be provided on the base end side of the light source unit 21 near the light source unit 21.

When the excitation light determination unit 60 is further provided on the base end side of the fluorescent member 22 or 41 near the fluorescent member 22 or 41, the following effect is obtained. That is, it is possible to diagnose the deterioration state of the first optical transmission unit 24 or 53 which is interposed between the two excitation light determination units 60 by comparison of the determination results of the light amount by the excitation light determination units 60 provided at two places.

When the light determination unit 30 is provided on the base end side of the second optical transmission unit 54 and the front end side of the second optical transmission unit 54 near the fluorescent member 22 or 41, the following effect is obtained. That is, it is possible to diagnose the deterioration state of the second optical transmission unit 54 which is interposed between the two light determination units 30 by comparison of the determination results of the light amount by the light determination units 30 provided on the base end side and the front end side of the second optical transmission unit 54.

When the excitation light determination unit 60 is provided on the base end side near the fluorescent member 22 or 41, and the light determination unit 30 is provided on the front end side near the fluorescent member 22 or 41, the following effect is obtained. That is, it is possible to diagnose the deterioration state of the fluorescent member 22 or 41 which is interposed between the excitation light determination unit 60 and the light determination unit 30 by comparison of the determination results of the light amount by the excitation light determination unit 60 and the light determination unit 30 provided at two places.

When the excitation light determination unit 60 is provided on the front end side near the light source unit 21, and the light determination unit 30 is provided on the front end side near the fluorescent member 22 or 41, the following effect is obtained. That is, it is possible to compare the amount of excitation light emitted from the light source and the amount of illumination light emitted from the fluorescent member 22 or 41 by comparison of the determination results of the light amount by the excitation light determination unit 60 and the light determination unit 30 provided at two places.

It is also possible to diagnose the deterioration state of the entire illumination device 1a or 50a by comparison of the excitation light determination unit 60 provided near the light source unit 21 and video output to the monitor 4.

In the illumination device 50a, the fluorescent member 41 and the first optical transmission unit 24 or 53 may be formed as a single body.

That is, a case is considered where a fluorescent material 80 of the fluorescent member 41 in FIG. 18 is distributed so as to be segregated on the front end side of resin 70 constituting the fluorescent member 41 as shown in a portion A of FIG 18. That is, when the fluorescent material 80 of the fluorescent member 41 has a concentration distribution such that the content ratio increases from the base end side toward the front end side in the fluorescent member 41, in the resin 70, a portion having a relatively low content ratio of the fluorescent material 80 can be regarded as the first optical transmission unit 24 or 53. The portion having a relatively low content ratio of the fluorescent material 80 is the base end side (a portion B of FIG. 18) of the fluorescent member 41.

In this case, the first optical transmission unit 24 or 53 is inexpensive compared to a fiber made of quartz or the like. Even when the first optical transmission unit 24 or 53 is damaged due to external stress, the resin 70 having a Young's modulus lower than quartz is used as the first optical transmission unit 24 or 53, such that the resin 70 is deformed, thereby suppressing damage to the first optical transmission unit 24 or 53.

In the illumination devices 1a and 50a, the light source unit 21 may be an LED.

In this case, in particular, it is possible to detect degradation in translucency due to deterioration of sealing resin constituting an LED, and an LED is inexpensive and has a long lifetime in comparison to an LED or a halogen lamp. With the use of an LED, it is possible to reduce complexity of temperature management compared to the related art.

### Industrial Applicability

According to the illumination device and the endoscope apparatus of the embodiments of the invention, since the light determination unit and the excitation light determination unit are provided, when excitation light is irradiated from the light source unit onto the fluorescent member such that illumination light is emitted from the fluorescent member and is irradiated to the outside, it is possible to quantitatively evaluate the amount of illumination light accurately.

## Claims

1. An endoscope apparatus (50) comprising:
an insertion portion (2) having a front end portion (10) having a front end surface (10a), a bending portion (11), and a tube portion (12), the bending portion (11) being located between the front end portion (10) and the tube portion (12); and an illumination device (50a) comprising:
a light source unit (21) which is configured to emit excitation light;
a first optical transmission unit (53) which is configured to guide excitation light from the light source unit (21);
a fluorescent member (22, 41) which is located in the tube portion (12) and is configured to be excited by excitation light to emit illumination light;
an illumination light determination unit (30) which is configured to determine the amount of illumination light emitted from the fluorescent member (22, 41) to output a determination signal; and
a second optical transmission unit (54) which is configured to guide illumination light from the fluorescent member (22, 41) to the front end surface (10a) to be irradiated outside the endoscope apparatus (50), and
an excitation light determination unit (60) which is configured to determine the amount of excitation light emitted from the light source unit (21), and has a first light sensor (60a) and a second light sensor (60b), the first light sensor (60a) being a photodiode configured to determine the amount of light substantially having the same wavelength as laser light in excitation light, and the second light sensor (60b) being a photodiode configured to determine the amount of light having a wavelength other than
the wavelength of laser light in excitation light,
wherein the illumination light determination unit (30) is configured to determine the amount of illumination light guided by the second optical transmission unit.

## Patentansprüche

1. Endoskopgerät (50), das umfasst:
einen Einführabschnitt (2), der einen vorderen Endabschnitt (10) mit einer vorderen Endoberfläche (10a), einen Biegeabschnitt (11) und einen Röhrenabschnitt (12) hat, wobei der Biegeabschnitt (11) zwischen dem vorderen Endabschnitt (10) und dem Röhrenabschnitt (12) angeordnet ist; und
eine Beleuchtungseinrichtung (50a), die umfasst:
eine Lichtquelleneinheit (21), die dazu eingerichtet ist, Anregungslicht zu emittieren;
eine erste optische Transmissionseinheit (53), die dazu eingerichtet ist, Anregungslicht von der Lichtquelleneinheit (21) zu führen;
ein fluoreszentes Element (42, 41), das in dem Röhrenabschnitt (12) angeordnet und dazu eingerichtet ist, durch das Anregungslicht angeregt zu werden, um Beleuchtungslicht zu emittieren;
eine Beleuchtungslichtbestimmungseinheit (30), die dazu eingerichtet ist, die Menge des von dem fluoreszenten Elements (22, 41) emittierten Beleuchtungslichts zu bestimmen, um ein Bestimmungssignal auszugeben; und
eine zweite optische Transmissionseinheit (54), die dazu eingerichtet ist, Beleuchtungslicht von dem fluoreszenten Element (22, 41) zu der vorderen Endoberfläche (10a) zu führen, um außerhalb des Endoskopgerät (50) abgestrahlt zu werden, und
eine Anregunglichtbestimmungseinheit (60), die dazu eingerichtet ist, die Menge des von der Lichtquelleneinheit (21) emittierten Anregungslichts zu bestimmen und die einen ersten Lichtsensor (60a) und einen zweiten Lichtsensor (60b) hat, wobei der erste Lichtsensor (60a) eine Fotodiode ist, die dazu eingerichtet ist, die Lichtmenge in dem Anregungslicht zu bestimmen, die im Wesentlichen die gleiche Wellenlänge wie Laserlicht hat, und wobei der zweite Lichtsensor (60b) eine Fotodiode ist, die dazu eingerichtet ist, die Lichtmenge in dem Anregungslicht zu bestimmen, die eine sich von der Wellenlänge von Laserlicht unterscheidende Wellenlänge hat,
wobei die Beleuchtungslicht Bestimmungseinheit (30) dazu eingerichtet ist, die durch die zweite optische Transmissionseinheit geführte Lichtmenge zu bestimmen.

## Revendications

1. Appareil (50) d'endoscope comprenant :
une partie d'insertion (2) comportant une partie d'extrémité avant (10) comportant une surface d'extrémité avant (10a), une partie de courbure (11) et une partie de tube (12), la partie de courbure (11) étant placée entre la partie d'extrémité avant (10) et la partie de tube (12) ; et
un dispositif d'éclairage (50a) comprenant :
une unité de source de lumière (21) qui est configurée pour émettre une lumière d'excitation ;
une première unité de transmission optique (53) qui est configurée pour guider la lumière d'excitation depuis l'unité de source de lumière (21) ;
un élément fluorescent (22, 41) qui est placé dans la partie de tube (12) et est configuré pour être excité par la lumière d'excitation pour émettre la lumière d'éclairage ;
une unité (30) de détermination de lumière d'éclairage qui est configurée pour déterminer la quantité de lumière d'éclairage émise depuis l'élément fluorescent (22, 41) pour délivrer en sortie un signal de détermination ; et
une deuxième unité de transmission optique (54) qui est configurée pour guider la lumière d'éclairage de l'élément fluorescent (22, 41) à la surface d'extrémité avant (10a) devant être irradiée à l'extérieur de l'appareil (50) d'endoscope, et
une unité (60) de détermination de lumière d'excitation qui est configurée pour déterminer la quantité de lumière d'excitation émise depuis l'unité de source de lumière (21), et comporte un premier capteur de lumière (60a) et un deuxième capteur de lumière (60b), le premier capteur de lumière (60a) étant une photodiode configurée pour déterminer la quantité de lumière présentant sensiblement la même longueur d'onde qu'une lumière laser dans la lumière d'excitation, et le deuxième capteur de lumière (60b) étant une photodiode configurée pour déterminer la quantité de lumière présentant une longueur d'onde autre que la longueur d'onde de la lumière laser dans la lumière d'excitation,
dans lequel l'unité (30) de détermination de lumière d'éclairage est configurée pour déterminer la quantité de lumière d'éclairage guidée par la deuxième unité de transmission optique.
